# EUROPEAN PATENT SPECIFICATION

(11) **EP 0 557 244 B1**
(45) Date of publication and mention of the grant of the patent: **13.03.1996**
(21) Application number: 93810066.6
(22) Date of filing: 03.02.1993
(51) Int. Cl.: A61K 31/44, A61K 9/20

(54) **Dosage forms having prolonged active-ingredient release**
Darreichungsformen mit verlaengerter Wirkstoffreigabe
Applications de l'efficacité à longue spectre

(30) Priority: 17.02.1992 CH 469/92
(43) Date of publication of application: 25.08.1993
(73) Proprietor: Siegfried Pharma AG, CH-4800 Zofingen (CH)
(72) Inventor: Compassi, Sabine, CH-6362 Stansstad (CH)
(74) Representative: Arnold, Winfried

(56) References cited:
- EP-A- 0 137 198
- EP-A- 0 168 044
- EP-A- 0 220 760
- EP-A- 0 274 176
- EP-A- 0 324 982
- EP-A- 0 339 420
- WO-A-89/02738
- CH-A- 643 455
- US-B- 4 389 393

## Description

### FIELD OF THE INVENTION

The invention relates to solid, orally administrable pharmaceutical dosage forms having prolonged active ingredient release for once-daily administration, containing calcium antagonists of the dihydropyridine type as active ingredients.

### BACKGROUND OF THE INVENTION

Calcium antagonists of the dihydropyridine type and their uses, for example as cardiovascular agents, are known (see British Patent 1 173 862; British Patent 1 358 951; US Patent 42 56 749; DE-OS 33 11 003). Those compounds, such as nifedipine, which is one of the most well known representatives of the group, are used especially for the treatment of coronary heart disease, for the prophylaxis of attacks of Angina pectoris and for the treatment of hypertension.

Various dosage forms are known which, depending upon the galenical characteristics, are suitable in various forms for the treatment of the above indications. Attention should be paid especially also to the physico-chemical properties of the substance. Nifedipine and other calcium antagonists of the dihydropyridine type are only very slightly voluble in water. For example, a maximum of 10 mg of nifedipine is soluble in 1000 ml of simulated gastric or intestinal fluid (0.001 %). Solubilities of less than 0.3 % in aqueous media are likely to give rise to absorption problems which manifest themselves in a reduction in the rate of absorption and in the amount absorbed (Barker et al.; Austral. J. Pharm. 49, 33-43, 1968).

For example, crystalline nifedipine is absorbed so slowly that the pharmacokinetic elimination rate exceeds the absorption rate. In comparison with capsules containing nifedipine in dissolved form, this results in plasma levels that are relatively low but fall more slowly (so-called Flip-Flop; Wagner JG., Fundamentals of Clinical Pharmacokinetics, Hamilton, III. 1975, Drug Intelligence Publications). For example, according to G. Pabst et al., Arzneim.-Forsch./Drug Res. 36(1), 256-260, 1986, after the administration of 20 mg of dissolved nifedipine in capsules, plasma level peaks of about 200 ng/ml are found as early as after about 30 minutes. After the administration of the same dose of crystalline nifedipine, the plasma level peaks after about 1.5 hours are about 40 ng/ml. Nifedipine dissolved in capsules therefore achieves plasma levels that are high but fall rapidly, whereas the administration of the same dose in crystalline form in tablets results in plasma levels that are lower but longer lasting.

Nifedipine in capsules is used especially when an immediate effect is required (treatment of an attack of Angina pectoris, treatment of hypertensive crises). The dosage is then 3 x 5 mg, 3 x 10 mg or 3 x 20 mg daily. However, the rapid rate of nifedipine influx increases the risk regarding reflex tachycardia.

In order to attain and maintain constant levels of active ingredients in the plasma there are used, on the one hand, infusion solutions, which are, however, unsuitable for ambulant treatment, or, on the other hand, retard preparations from which the active ingredient is released only in a delayed manner into the biological system.

Nifedipine in crystalline form is therefore especially suitable, for example, for long-term ambulant treatment of hypertension or coronary heart disease. The most customary dosage is 2 x 20 mg daily. In many cases the dosage has to be increased to 2-3 x 40 mg per day. The solubility behaviour and thus also the absorption rate can be controlled to a certain degree via the crystal size of nifedipine (EP 47 899).

For the manufacture of retard preparations it is also possible to use amorphous nifedipine by controlling the good solubility of the non-crystalline form using suitable excipients (EP 232 155; EP 220 760; DE-OS 30 24 858). Several possible methods of manufacturing amorphous nifedipine preparations having improved solubility behaviour on the basis of the molecularly disperse presence of the substance have been disclosed (cf. DE-OS 28 22 882). The preparation of amorphous nifedipine generally requires organic solvents, there being used especially methylene chloride because of its excellent dissolving power. Where possible, however, chlorinated hydrocarbons should be avoided in the manufacture of modern medicaments. Ethanol is a less efficient solvent, since nifedipine is much less soluble therein and therefore large amounts of solvent are required. In addition, in the amorphous state the material is generally unstable and may change into the more stable crystalline form again, for which triggering factors include heat and moisture.

Known pharmaceutical preparations having delayed release of nifedipine, for example AdalatR retard or CorotrendR retard, involve especially twice daily administration and, as in vitro tests show, release 60 - 90 % of the total dose of the medicinal substance within 8 hours (see Table 1). The lowest plasma levels regarded as still being effective, approximately 10 - 15 ng/ml, are attained as early as 6 to 8 hours after the administration of 20 mg (see Pabst and EP 220 760). When a tablet containing 40 mg of nifedipine is used, the plasma levels after 12 hours fluctuate above the necessary minimum effective concentration (EP 220 760), but the 40 mg form described rapidly attains plasma level peaks of more than 60 ng/ml initially which then, in accordance with the specific elimination kinetics (Flip-Flop model), fall to about 20 - 25 ng/ml within the first 9 hours. After 16 hours the concentrations are about 15 - 17 ng/ml and after 24 hours they are still about 8 - 11.4 ng/ml. That plasma level behaviour is not optimum for once-daily administration, since in order to obtain plasma levels above the minimum effective threshold during the second twelve hours it is necessary to accept high plasma levels during the first 12 hours. As mentioned above, the rapid influx rate of nifedipine in conjunction with high plasma level peaks has repeatedly been associated with an increased side-effect rate (tachycardia) and reduced effectiveness in lowering blood pressure (Kleinbloessem et al.; Clin. Pharmacol. Ther. 35,6, 742-749, 1984).

Accordingly, it would be desirable to have nifedipine formulations that are distinguished by a slow influx rate and small plasma level fluctuations, that is to say plasma levels that remain constant over a relatively long period. That medicament should provide, as early as on the first administration or on repeated administration, constant, therapeutically effective plasma levels exhibiting a minimum of fluctuations between the maximum and minimum concentrations of active ingredient in the blood. A possible method of reducing the influx time of the active ingredient and of minimising fluctuations lies in controlling the dissolution of the active ingredient over an even longer period of time than is the case with conventional retard formulations. That requires the active ingredient to be absorbed over the entire gastro-intestinal tract.

A solution to this problem is offered by the therapeutic system OROS^{R} (F. Theeuwes, J. Pharm. Sci., Vol. 64,12, 1987-1991, 1975) which has already been described for sparingly soluble active ingredients with a double chamber system (USP 4 111 202) and especially for nifedipine (BE 898 819). It can be seen from Table 1 that the system containing 30 mg of nifedipine releases only about 20 % of the total dose after the first 8 hours. The liberation rate from the third hour is linear, that is to say about 3.33 - 4 % (i.e. 0.9 - 1.2 mg) of the total dose are released per hour. That release principle differs quite clearly from the dissolution curves of conventional retard forms for twice-daily administration in which after 8 hours 60 -100 % of the dose, generally 20 mg, are released with a non-linear profile (Table 1). Using the OROS therapeutic system it is possible with 30 mg of nifedipine to maintain plasma levels of approximately 10 - 20 ng/ml over a period of 24 hours without the necessity to accept plasma level peaks. A disadvantage of the OROS systems is that they are technically difficult to produce.

The release rate from tablets or powders is influenced by the solubility characteristics of the active ingredient which, in turn, depend upon particle size, specific surface area and interactions with excipients. Dissolution can be retarded by means of diffusion barriers in the core of the tablet or in a film coating. Retarding dissolution by means of diffusion barriers in the core is a principle that is frequently used on account of its technical simplicity. It is possible to use various excipients, for example swelling agents, lipophilic substances or alternatively plastics, as diffusion barriers. The matrix, that is to say the homogeneous substance composition, can be such that the release of the active ingredient takes place by diffusion of the dissolved active ingredient especially through the water-filled pores in the tablet core and if required in special cases by diffusion through the retarding substance which must for that purpose be in a suitable structural form. Alternatively the matrix also can be in a form that is subjected to slow erosion and in this way effects delayed release of the active ingredient.

In all those cases the diffusion path and the active diffusion surface for the release change with time. For that reason it is clear that with matrix systems neither in vivo nor in vitro is it usually possible to expect any release having linear kinetics, that is to say of the 0^{th} order. Instead, the release is generally a function of the root of the time (Square root dissolution; Higuchi; J. Pharm. Sci. 52,12,1963, 1145). The validity of the Higuchi law for the hydrocolloid matrix has also been documented in numerous publications (Ford et al., Int. J. Pharm., 24, 1985, 327-338; 339-350; 1985).

Therapeutic dosage forms in which the medicinal substance is incorporated into a soluble or erodible matrix would be desirable per se on account of the ease of their manufacture, the low degree of variation between different manufacturing processes and because of the relatively low costs.

The use of hydrophilic gums, such as hydroxypropylmethylcellulose, as delaying matrix material is known and has been tested with a large number of active ingredients, but no formulation has been disclosed hitherto that would be suitable for attaining the desired objectives with calcium antagonists of the dihydropyridine type, such as nifedipine.

The behaviour of a specific medicinal substance when combined with a retarding excipient cannot be calculated or generally predicted. Although the basic factors affecting release from matrix systems have been well researched, interactions between the retarding material on the one hand and the active ingredient and other excipients on the other can affect the retarding action in various ways.

In particular, the manufacture of monolithic matrix forms having a release profile according to the 0^{th} order is one of the important problems of galenical pharmacy. Long-term release systems that obey the Higuchi law of release are disadvantageous because the release rates decline markedly with time.

A release rate of the 0^{th} order is difficult to produce because, as mentioned, for geometric reasons lengths of diffusion path for the active ingredient that are dependent upon time and rate of release have to be overcome. The release rate decreases as the length of the diffusion path increases, that is to say as time passes less and less substance is released.

It is therefore remarkable that in certain cases and under favourable conditions both readily soluble and sparingly soluble medicinal substances exhibit a linear dissolution principle from matrix systems containing hydroxypropylmethylcellulose (HPMC) (Ranga Rao et al., Drug Development and Industrial Pharmacy, 14 (15-17), 2299-2320, 1988 and Ranga Rao et al., J. of Controlled Release, 12, 1990, 133-141). The solubility of the medicinal substance is therefore not absolutely critical for release of the 0^{th} order.

The question of release kinetics is a multi-factored problem in which, in addition to the dissolution properties of the active ingredient, a part is played by the rate of water absorption and thus the rate of swelling of the interface to be penetrated, the diffusion coefficient of the substance through the swollen mass and also the time-dependent thickness thereof. It can clearly be imagined that release of the 0^{th} order is brought about by the existence of an equilibrium between the erosion of the tablet and the dissolution of the active ingredient, so that the diffusion paths for the substance remain constant over the dissolution time. Such a pharmaceutical dosage form cannot be prepared without inventive activity.

Published data of S. Leucuta et al. (Pharmazie, 43, 1988, 845 ff) show, in addition, that the Higuchi release kinetics are observed in the case of a nifedipine/HPMC system under customary conditions.

### PROBLEM OF THE INVENTION

The problem underlying the present invention is to overcome the existing prejudices by developing a solid, orally administrable pharmaceutical dosage form for sparingly water-soluble calcium antagonists that are absorbable over the entire gastro-intestinal tract, especially for nifedipine, that is technically easy to produce and is suitable for maintaining a constant, therapeutically effective level of active ingredient in the plasma over a period of approximately 24 hours with a once-daily oral administration. This represents the development of a medicament in which release is delayed to a greater extent than in conventional retard forms (Adalat retard 20), there being sought in vivo a release profile that is as linear as possible, that is to say a therapeutic medicament to be characterised over a period of several hours essentially by a dissolution process of the 0^{th} order. Since the active ingredient, once dissolved in the gastro-intestinal tract, is absorbed rapidly and since only the kinetics of the release will be the rate-limiting factor for absorption, a constant rate of release in vivo could maintain the absorption rate at a constant value. As a result of this dissolution process of the 0^{th} order, it is possible to achieve infusion-like pharmacokinetic conditions. Conversely, infusion-like pharmacokinetic conditions after the administration of a medicament indicate an absorption rate of the 0th order or, in the case of release-limited absorption, an in vivo release of the 0^{th} order.

As mentioned above, a constant rate of release of the 0^{th} order from solid dosage forms, such as tablets or powder capsules, is not what would immediately be expected of sparingly soluble active ingredients and is difficult to achieve since many parameters must be taken into account.

It can be seen from the above comments that release kinetics of the 0^{th} order are not to be expected a priori with sparingly soluble calcium antagonists of the dihydropyridine type, such as nifedipine, with HPMC systems. Release kinetics of the 0^{th} order are to be regarded as a special case which may occur only with certain dosage forms. From the large number of known pharmaceutical excipients it is necessary to select those suitable for the desired purpose and to process them in suitable quantity ratios, which must likewise be selected, to form a matrix system. Such dosage forms are provided by the formulations according to the invention.

### DETAILED DESCRIPTION OF THE INVENTION

The invention relates to a pharmaceutical dosage form having prolonged release of zero order of the active ingredient, suitable for maintaining a therapeutically effective plasma level over a period of 24 hours with a once-daily oral administration, containing as active ingredient a therapeutically effective amount of a crystalline, sparingly water-soluble calcium antagonist of the dihydropyridine type,
characterised by
a homogeneous matrix containing 5 - 60 % by weight of a crystalline, sparingly water-soluble calcium antagonist of the dihydropyridine type having a specific surface area of from 0.2 to 0.5 m²/g,
2 - 50 % by weight of hydroxypropylmethylcellulose having a molecular weight in the range of about 20,000 - 250,000,
optionally 2 - 25 % by weight of pharmaceutically acceptable excipients controlling release, and
optionally other pharmaceutically acceptable excipients making up the weight of the dosage form to 100 %.

### Short description of the Figures:

Figure 1 shows the percentage of release of nifedipine up to 8 hours in vitro in simulated intestinal fluid (sif) from the tablet according to Example 8.

Figure 2 shows the percentage of release of nifedipine up to 24 hours in vitro in simulated intestinal fluid (sif) from the tablet according to Example 8.

Figure 3 shows the correlation of the in vivo absorption rate up to 24 hours, calculated according to Wagner-Nelson, J. pharm. Sci. 52, Vol.6, 1963, page 610-611,(_·_) and the in vitro dissolution rate in simulated intestinal fluid of the nifedipine tablet according to Example 8 during 8 (__{□}_) and 24 hours (_x_).

The curves are of 0^{th} order and indicate the good in vitro/in vivo correlation. In vitro dissolution results are obtained by the method of Langenbucher et al., Pharm. Ind. 51, 11, 1989, page 1276 - 1281. The media used are simulated intestinal fluid (sif) of pH 7.2 ± 0.2, containing 8.05 g of disodium hydrogenphosphate, 1.56 g of disodium dihydrogenphosphate and demineralised water up to 1000 ml, and simulated gastric fluid containing 2.0 g of sodium chloride, 80.0 ml of 1N hydrochloric acid and demineralised water up to 1000.0 ml.

Pharmaceutical dosage forms are especially tablets and powders introduced into capsules, for example hard gelatin capsules.

Prolonged release of the active ingredient is to be understood as being especially a rate of release over a period of approximately 24 hours that increases slowly at first and is then substantially constant per unit of time over a period of several hours (release of the 0^{th} order).

A therapeutically effective plasma level is to be understood as being more than 10 ng of active ingredient, for example nifedipine, per ml of plasma.

A therapeutically effective amount is the amount required for maintaining the desired therapeutic effect over a period of approximately 24 hours, that is to say from 20 to 120 mg of active ingredient, preferably 20, 30, 40, 50, 60, 70, 80, 90, 100 or 120 mg.

Sparingly water-soluble calcium antagonists of the dihydropyridine type are those which have approximately the same water-solubility as nifedipine, that is to say that are about 0.001 % soluble, or alternatively are slightly less or more soluble, for example approximately from 0.0001 to 0.01 % soluble, in aqueous, simulated gastric or intestinal fluid. Special mention should be made of nifedipine and also of nitrendipine. Other calcium antagonists of this type are, for example, nimodipine, isradipine, nicardipine, niludipine, nigludipine, nisoldipine, felodipine, amlodipine and lacidipine. According to the invention, those active ingredients are in crystalline form. The specific surface area thereof is, for example, approximately 0.2 - 0.5 m²/g (BET), preferably 0.3 - 0.4 m²/g.

A matrix is defined in galenical pharmacy as being a well mixed, homogeneous substance composition that can be compressed to form tablets or can be introduced in the form of a powder into capsules, in this instance preferably hard gelatin capsules.

The tablets or capsules contain, for example, 20 - 120 mg, preferably 20, 30, 40, 50, 60, 70, 80, 90, 100 or 120 mg, of active ingredient, for example nifedipine or alternatively nitrendipine.

The hydroxypropylmethylcellulose (HPMC) used according to the invention represents the retarding principle, is preferably of the 2208 USP XXII type, has a molecular weight of 20,000 - 250,000, preferably 20,000 - 120,000, and has a preferred viscosity of 100 - 100,000, preferably 100 - 15,000 cps. Especially suitable are Methocel K types which produce the fastest swelling, for example Methocel K100LV, Methocel K4M and Methocel K15M (brand names, DOW CHEMICAL CO.) or the virtually equivalent Metolose 90SH types, for example Metolose 90SH100, Metolose 90SH4,000 and Metolose 90SH15,000 (brand names, Shin-Etsu Chemical Co. Ltd.). Approximately 2 - 50 % by weight HPMC are used, based on the final weight of the tablet or capsule filling.

The pharmaceutically acceptable excipients controlling release are lipophilic or hydrophilic substances (release retarders, liberation controllers) that modify the swelling process of the retarding matrix. Hydrophilic release retarders are solid polyethylene glycols, for example polyethylene glycol 4,000 or 6,000, or polyvinyl-pyrrolidones, for example Kollidone 25, Kollidone 30 or Kollidone 90 (brand names of BASF GmbH) having various viscosities, and also vinylpyrrolidone/vinyl acetate copolymers, for example Kollidone VA 64 (brand name BASF GmbH). Lipophilic release retarders are pharmaceutically acceptable derivatives of vegetable fats in solid, tablet-table form having a melting point of above 60°, such as vegetable fatty acids having chain lengths of at least 16 carbon atoms, for example stearic acid C16, palmitic acid C18 or mixtures thereof, and especially vegetable oils hardened by hydrogenation, for example hydrogenated castor oil, such as Cutina HR (brand name of Henkel) or hydrogenated cottonseed oil, such as Emvelop or Lubritab (brand names of Mendell). For the preparation of tablets the lipophilic release retarders must be suitable for tabletting.Optionally 2 - 25 % release retarder are used, based on the final weight of the tablet or capsule and in dependence of the nature of the retardation of release desired.

Further excipients are certain fillers, lubricants and flow-regulating agents, which may likewise exert an effect, albeit a small one, on the release kinetics.

Fillers are corn starch, lactose, powdered and microcrystalline cellulose, mannitol or dicalcium phosphate, and also mixtures thereof. Preferred is a mixture of 75 % lactose and 25 % powdered cellulose, for example Cellactose (brand name of Meggle GmbH). The fillers must therefore be carefully selected in suitable amounts and matched exactly to the specific formulation. In addition, attention should be paid to the compression properties. They are used in an amount making up the weight of the tablet to 100 %.

Lubricants are, for example, magnesium stearate, stearic acid of a suitable quality, calcium stearate, and mixtures thereof, magnesium stearate being preferred, and are preferably used in an amount of 0.2 - 1 %, based on the final weight of the formulation. Suitable agents that act on the flowability of the powder to be encapsulated or compressed (flow-regulating agents) are, for example, highly dispersed silicon dioxide, preferably in an amount of 0.25 - 1 %, based on the final weight of the formulation.

The tablets can be provided with a neutral film coating or with a film coating that delays the release of the active ingredient, that is to say that produces a lag time.

A film coating having no retarding action consists, for example, of film-formers, pigments, anti-adhesive agents and plasticisers. Such a film former may consist of fast-dissolving constituents in which case it is preferable to use low-viscosity hydroxypropylmethylcellulose type 2910 USP XXII, for example Methocel E5 or E 15 (Dow Chemicals Ltd.) or Pharmacoat 606 (Shin-Etsu).

A film coating having retarding action may consist of water-insoluble but water-permeable polymers which, as a diffusion barrier, not only bring about a lag time at the beginning but also affect the swelling behaviour of the core over a prolonged period as a result of the initially altered water permeation. Preferred water-insoluble polymers are water-insoluble derivatives of methacrylic acid, for example methyl/ethyl acrylate, such as Eudragit RS or RL and Eudragit NE (brand names, Röhm Pharma GmbH) and mixtures thereof. The dosage should be adapted to the retarding effect desired and is, for example, 1 - 1.5 mg/cm² surface area.

The film coating may also contain excipients customary in film-coating procedures, such as light-protective pigments, for example iron oxide, in an amount of approximately 40 - 80 %, or titanium dioxide, in an amount of 100 - 150 %, anti-adhesive agents, for example talc, in an amount of approximately 50 - 200 %, and also suitable plasticisers, matched to the polymer, of the polyethylene glycol series, for example PEG 400 or PEG 6,000, or triethyl citrate in the case of films based on methacrylic acid derivatives, such as Eudragit RS/RL and NE, in an amount of approximately 30 - 60 % (percentages are in each case based on the dry coating substance). When aqueous dispersions of the said Eudragit types are used, then, for example, Tween 80 is necessary as aggregation inhibitor.

For the preparation of the powder components for filling hard gelatin capsules it is possible to use the same powder components as those used for the preparation of tablets; surprisingly the same release profiles are achieved.

The release kinetics in tablets are also dependent upon geometric factors, such as the shape and size of the tablets. Biconvex tablets having a diameter of approximately 5 - 11 mm, especially 7 - 9 mm, and a thickness of 3 - 5 mm, especially 4 mm, are preferred.

Preferred tablets contain, for example:
- 40 mg of nifedipine or alternatively nitrendipine,
- approximately 2 - 35 mg of hydroxypropylmethylcellulose type 2208 USP XXII of 100 or 4000 cps,
- approximately 20 - 50 mg of lactose and 35 to 60 mg of powdered and/or microcristalline cellulose, or instead approximately 50 - 80 mg of a mixture of 75 % lactose and 25 % powdered cellulose (CellactoseR, Meggle),
- optionally approximately 10 mg of hardened vegetable oil,
- approximately 1.5 mg of magnesium stearate,
- optionally 0,25 - 1 mg of highly dispersed silicon dioxide, so that cores prepared therefrom weigh 140 - 155 mg, and are optionally provided with a coating containing
- approximately 1.5 - 2.5 mg of titanium oxide, and
- approximately 0.5 - 1.5 mg of red iron oxide,
- approximately 1 - 3.0 mg of talc,
- optionally approximately 2.0 mg of hydroxypropylmethylcellulose type 2910 having a viscosity of 5 to 15 cps,
- optionally 1 - 1.5 mg of polyethylene glycol 400,
- optionally approximately 0.005 mg of polysorbate 80,
- optionally approximately 0,5 - 0.75 mg of triethyl citrate,
- optionally approximately 0.5 - 1.5 mg of Eudragit RL dry substance, and/or
- optionally approximately 0.5 - 1.5 mg of Eudragit RS dry substance.

For tablets containing 20, 30, 50, 60, 70, 80, 90, 100 and 120 mg of active ingredient, corresponding aliquots of the excipients should be used.

Preferred pharmaceutical dosage forms are those in which the constant rate of release of active ingredient is about 0.1 - 4 mg/h, especially about 1 - 3.5 mg/h, for the dosage form containing 40 mg, and specifically the tablets described in the Examples, in particular Examples 3, 5, 7 and 8.

The release rates of the tablets according to Examples 1 to 7 in simulated gastric fluid, determined by the through-flow method (1 litre/h) and HPLC analysis of the nifedipine released, are shown in Table 1. For comparison purposes, the Table shows the release rates, determined in the same way, for the commercially available, conventional retard formulations Adalat retard 20 mg and Nifhexal retard 40 mg, and also for the formulations of nifedipine that are suitable for once-daily administration, the OROS formulations Procardia XL 30 mg and XL 60 mg.

**Table 1**

| Rates of release (mg/h) in simulated gastric fluid | | | | | | | | | |
|---|---|---|---|---|---|---|---|---|---|
| time h | 0 | 1 | 2 | 3 | 4 | 5 | 6 | 7 | 8 |
| Adalat 20 mg | 0.0 | 4.4 | 2.4 | 1.8 | 1.2 | 1.4 | 1.0 | 1.0 | 0.8 |
| Nifhexal 40 mg | 0.0 | 6.0 | 6.0 | 4.6 | 3.6 | 2.7 | 2.3 | 1.8 | 1.6 |
| Procardia XL 30 mg | 0.0 | 0.0 | 0.0 | 0.0 | 1.0 | 0.7 | 1.0 | 1.4 | 1.3 |
| Procardia XL 60 mg | 0.0 | 0.0 | 0.0 | 0.5 | 1.3 | 2.0 | 2.3 | 2.5 | 2.6 |
| Example 1 40 mg | 0.0 | 1.1 | 2.1 | 2.7 | 3.0 | 3.6 | 3.8 | 3.5 | 3.2 |
| Example 2 40 mg | 0.0 | 0.0 | 0.2 | 0.4 | 0.8 | 0.8 | 1.6 | 1.4 | 1.6 |
| Example 3 40 mg | 0.0 | 0.4 | 0.7 | 0.9 | 1.4 | 1.7 | 2.1 | 2.6 | 2.6 |
| Example 4 40 mg | 0.0 | 0.7 | 1.1 | 1.5 | 1.5 | 1.4 | 1.4 | 1.4 | 1.3 |
| Example 5 40 mg | 0.0 | 0.6 | 1.1 | 1.2 | 1.4 | 1.4 | 1.4 | 1.4 | 1.4 |
| Example 6 40 mg | 0.0 | 1.1 | 1.2 | 1.2 | 1.2 | 1.2 | 1.2 | 1.3 | 1.3 |
| Example 7 40 mg | 0.0 | 1.0 | 0.9 | 1.3 | 1.2 | 1.4 | 1.4 | 1.4 | 1.4 |

Table 1a shows, in mg, the total amounts of active ingredient released after 8 hours in % of the initial dose.

**Table 1a**

| Amount released after 8 hours in simulated gastric fluid | | | |
|---|---|---|---|
| | | % | mg |
| Adalat | 20 mg | 70 | 14.0 |
| Nifhexal | 40 mg | 72 | 28.6 |
| Procardia XL | 30 mg | 18 | 5.5 |
| Procardia XL | 60 mg | 19 | 11.2 |
| Example 1 | 40 mg | 58 | 23.0 |
| Example 2 | 40 mg | 17 | 6.8 |
| Example 3 | 40 mg | 31 | 12.4 |
| Example 4 | 40 mg | 26 | 10.3 |
| Example 5 | 40 mg | 25 | 9.9 |
| Example 6 | 40 mg | 24 | 9.7 |
| Example 7 | 40 mg | 25 | 10.0 |

The mean values, found using the tablets according to Examples 3 and 5, for plasma levels after a single oral administration in comparison with those of Oros system Procardia XL 30 mg and Adalat retard 40 mg (2 x 20 mg) are shown in Table 2:

**Table 2**

| Mean values (ng/ml) for plasma levels after a single oral administration | | | | |
|---|---|---|---|---|
| time h | Example 5 40 mg | Example 3 40 mg | Procardia XL 30 mg | Adalat retard 40 mg (2x20) |
| 0.0 | 0.00 | 0.00 | 0.00 | 0.00 |
| 0.5 | 3.15 | 2.05 | 0.00 | 24.09 |
| 1.0 | 5.49 | 5.61 | 0.00 | 37.74 |
| 2.0 | 11.43 | 10.43 | 0.24 | 49.21 |
| 3.0 | 17.06 | 17.91 | 4.86 | 54.50 |
| 4.0 | 15.38 | 18.16 | 6.61 | 43.20 |
| 6.0 | 13.30 | 17.65 | 11.21 | 32.63 |
| 8.0 | 13.39 | 17.53 | 12.46 | 25.33 |
| 10.0 | 12.35 | 15.88 | 13.54 | 20.98 |
| 12.0 | 12.45 | 14.25 | 16.34 | 17.24 |
| 24.0 | 12.83 | 11.69 | 13.86 | 8.09 |
| 36.0 | 4.41 | 3.4 | 5.33 | 1.81 |
| 48.0 | 2.01 | 2.75 | 1.41 | 0.66 |

The invention relates also to a process for the preparation of a pharmaceutical dosage form according to the above description, characterised in that the dosage form is prepared in a conventional manner.

The constituents of the tablet cores are, if necessary, ground to the desired particle size, mixed homogeneously with one another at the same time or in a specific sequence and, optionally, granulated by moistening with water, dispersing and drying the granular mass. If the mixture is granulated, the fillers, flow agents and lubricants can be added to the granules after granulation. The mixture of the core constituents is compressed to form tablets having a hardness of approximately 50 - 100 N, preferably 80 N, or is introduced as such into hard gelatin capsules.

The film-coating is effected in a conventional manner by mixing the constituents of the film coating with water, coating the compressed tablet cores therewith and drying at approximately from 30 to 40°C, preferably approximately 35°C.

The invention relates also to the use of a pharmaceutical dosage form in accordance with the present invention for the treatment of diseases that can be influenced by calcium antagonists, for example hypertension, comprising the once-daily oral administration of a pharmaceutical dosage form according to the present invention containing a therapeutically effective amount of a dihydropyridine derivative to a patient to be treated with calcium antagonists, for example a patient suffering from hypertension.

Depending upon the age and weight of the patient, the nature and severity of the illness as well as the general condition of the patient and also the dihydropyridine derivative to be administered, the dosage forms used contain 20, 30, 40, 50, 60, 70, 80, 90, 100, 110 or 120 mg of active ingredient.

The following Examples illustrate the invention but do not constitute a limitation thereof.

### Example 1: Tablets containing 40 mg of nifedipine

For the preparation of 22,000 tablets, 880 g of nifedipine having a specific surface area of 0.3 - 0.4 m²/g (BET), 440.0 g of HPMC 2208 100 cps (Methocel K100LV), 880.0 g of lactose and 533.5 g of microcrystalline cellulose (Avicel PH 101) are mixed together. The powder mixture is granulated with deionised water. The pre-dispersed and sieved (mesh size 1 mm) granular mass is dried for several hours at 40°C in vacuo and, if necessary, ground in a hammer mill and sieved again (mesh size 1 mm).

533.5 g of microcrystalline cellulose (Avicel PH 102) are then mixed in. For a ready-to-compress mixture, 33.0 g of magnesium stearate are also mixed in.

The granular mixture is compressed to form biconvex cores having a diameter of 7 mm, a thickness of 4 mm and a hardness of 80 N.

The release values in simulated gastric fluid are given in Table 1.

### Example 2: Film-coated tablets containing 40 mg of nifedipine

The cores from Example 1 are provided with a film coating using a mixture containing 70.0 g of talc, 50.0 g of titanium oxide, 22.5 g of red iron oxide, 0.125 g of polysorbate 80, 15.0 g of triethyl citrate, 82.5 g of Eudragit RL 30 D, 55.0 g of Eudragit RS 30 D and 990 g of water. The film-coated tablets are dried in a circulating air drying cabinet for at least 4 hours at 35°C.

The release rates in simulated gastric fluid are given in Table 1.

The release profile shows a pronounced lag time. Although the film coating disintegrates after about 1 - 2 hours, there is a marked reduction in the release rate in comparison with Example 1.

### Example 3: Film-coated tablets containing 40 mg of nifedipine

For the preparation of 22,000 tablets, 616.0 g of HPMC 2208 100 cps (Methocel K 100 LV), 220.0 g of hardened, hydrogenated vegetable oil, 22.0 g of silicon dioxide and 33.0 g of magnesium stearate are mixed together.

880.0 g of nifedipine having a specific surface area of 0.3 - 0.4 m2/g (BET) and 1529.0 g of a mixture containing 75 % lactose and 25 % powdered cellulose as a unitary product (Cellactose, Meggle) are added to the above powder mixture and mixed in.

The powder mixture is compressed to form tablets weighing 150 mg and having a diameter of 7 mm, a thickness of 4 mm and a hardness of 80 N.

The cores are provided with a film coating using a colloidal dispersion containing 50.00 g of hydroxypropylmethylcellulose (type 2910, viscosity 5 mPas), 27.50 g of polyethylene glycol 400, 50.00 g of titanium dioxide and 22.50 g of red iron oxide, 25.00 g of talc in 1375 g of water.

The film-coated tablets are dried in a circulating air drying cabinet for at least 4 hours at 35°C.

The release values of the cores and of the film-coated tablets in simulated gastric fluid are the same and are given in Table 1.

The mean values for the plasma levels over a period of 48 hours in eight human test subjects after administration of 40 mg are given in Table 2.

### Example 4: Hard gelatin capsules containing 40 mg of nifedipine

For the manufacture of 22,000 capsules, 616.0 g of HPMC 2208 100 cps (Methocel K 100 LV) and 880.0 g of nifedipine having a specific surface area of 0.3 - 0.4 m2/g are mixed together and granulated with deionised water.

The granular mass is dried in vacuo at temperatures of 40°C and ground in a hammer mill. 1529.0 g of a mixture containing 75 % lactose and 25 % powdered cellulose (commercially available unitary product Cellactose, Meggle), 220.0 g of hardened, hydrogenated vegetable oil, 22.0 g of silicon dioxide and 33.0 g of magnesium stearate are mixed into the granules.

The granules are introduced into hard gelatin capsules. The filling weight of the capsules is 150 mg. In order to protect the nifedipine against the action of light, the capsules contain pigments, such as iron oxide/titanium oxide.

The release values in simulated gastric fluid are given in Table 1.

### Example 5: Film-coated tablets containing 40 mg of nifedipine

For the preparation of 22,000 tablets, 440.0 g of HPMC 2208/4,000 cps (Methocel K4M or Metholose 90 SH 4,000), 880.0 g of nifedipine having a specific surface area of 0.3 - 0.4 m2/g (BET), 880.0 g of a commercially available unitary product consisting of 75 % lactose and 25 % powdered cellulose (Cellactose, Meggle) and 1067.0 g of microcrystalline cellulose are mixed together. For a ready-to-compress mixture, 33.0 g of magnesium stearate are added and the mixture is compressed to form tablets weighing 150 mg and having a diameter of 7.0 mm, a thickness of 4 mm and a hardness of 80 N.

The tablets are provided with the coating described in Example 3.

The release values of the cores and of the film-coated tablets in simulated gastric fluid are the same and are given in Table 1.

The mean values for the plasma levels over a period of 48 hours in eight human test subjects after administration of 40 mg are given in Table 2.

### Example 6: Film-coated tablets containing 40 mg of nifedipine

For the preparation of 22,000 tablets, 440.0 g of HPMC 2208/4,000 cps (Methocel K4M or Metholose 90SH-4,000) and 880.0 g of nifedipine having a specific surface area of 0.3 - 0.4 m2/g (BET) are granulated in accordance with Example 4.

880.0 g of a commercially available unitary product consisting of 75 % lactose and 25 % powdered cellulose (Cellactose, Meggle) and 1067.0 g of microcrystalline cellulose are mixed into the granules. For a ready-to-compress mixture, 33.0 g of magnesium stearate are added and the mixture is compressed to form tablets weighing 150 mg and having a diameter of 7.0 mm, a thickness of 4 mm and a hardness of 80 N.

The tablets are provided with the coating described in Example 3.

The release values of the cores and of the film-coated tablets in simulated gastric fluid are the same and are given in Table 1.

### Example 7: Hard gelatin capsules containing 40 mg of nifedipine

Hard gelatin capsules are filled with the powder mixture according to Example 5 or the granules according to Example 6. The filling weight of the hard gelatin capsules is 150 mg. The capsules contain pigments, such as iron oxide/titanium oxide, to protect the nifedipine against the action of light.

The release values are given in Table 1.

### Example 8: Film-coated tablets containing 40 mg of nifedipine

For the preparation of 900,000 tablets, 18 kg of HPMC 2208/4,000 cps (Methocel K4M or Metholose 90 SH 4,000), 36 kg of nifedipine having a specific surface area of 0.3 - 0.4 m²/g (BET), 36 kg of a commercially available unitary product consisting of 75 % lactose and 25 % powdered cellulose (Cellactose, Meggle) and 43.65 kg of microcrystalline cellulose are mixed together. For a ready-to-compress mixture, 1.35 kg of magnesium stearate and 0.675 kg of grinded colloidal silicon dioxide are separately added. The mixture is compressed to form tablets weighing 150.75 mg and having a diameter of 7.0 mm, a thickness of 4 mm and a hardness of about 70 - 80 N.

The tablets are coated with 1.8 kg of HPMC 2910/15 cps, 1.8 kg of titanium dioxide, 0.81 kg of red ferric oxide, 0.99 kg of polyethylene glycol 400, and 0.9 kg of talc, and polished with 0.07 kg of polyethylene glycol 6000.

The release values of the cores and of the film-coated tablets in simulated intestinal fluid are the same and are given in Table 3 for 8 hours and Table 3a for 24 hours.

**Table 3**

| Rates of release of nifedipine (mg/h and %) in simulated intestinal fluid during 8 h | | |
|---|---|---|
| time h | mg/h | % |
| 0 | 0.0 | 0.0 |
| 1 | 1.24 | 3.1 |
| 2 | 1.32 | 6.4 |
| 3 | 1.36 | 9.8 |
| 4 | 1.44 | 13.4 |
| 5 | 1.36 | 16.8 |
| 6 | 1.48 | 20.5 |
| 7 | 1.44 | 24.1 |
| 8 | 1.36 | 27.5 |

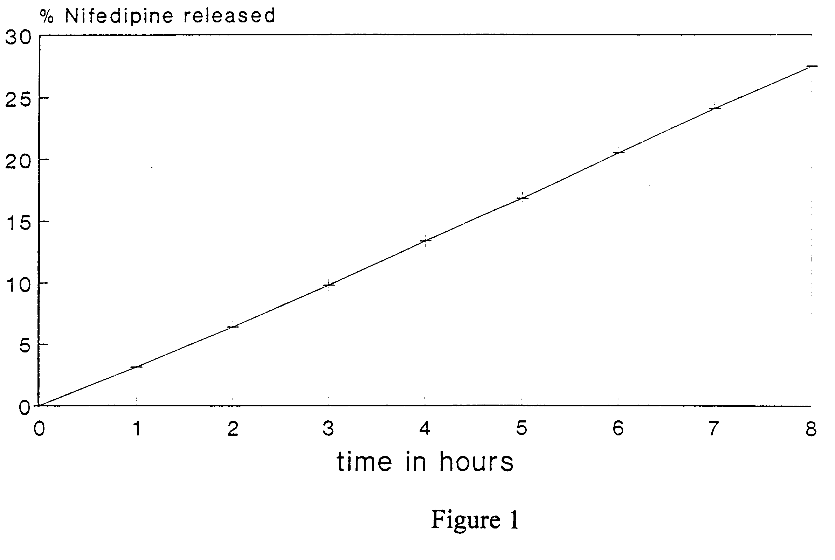

The percentages of release in simulated intestinal fluid of the Table 3 is represented by Figure 1 and is in form of a linear curve of 0^{th} order.

**Table 3a**

| Rates of release of nifedipine (mg/h and %) in simulated intestinal fluid during 24 h | | |
|---|---|---|
| time h | mg/h | % |
| 0 | 0.0 | 0.0 |
| 2 | 1.00 | 5.0 |
| 4 | 1.05 | 10.3 |
| 6 | 1.10 | 15.7 |
| 8 | 1.05 | 21.0 |
| 10 | 1.10 | 26.3 |
| 12 | 1.25 | 32.5 |
| 14 | 1.70 | 41.0 |
| 16 | 1.55 | 47.3 |
| 18 | 1.95 | 57.1 |
| 20 | 1.75 | 65.9 |
| 22 | 1.50 | 73.2 |
| 24 | 1.50 | 80.5 |

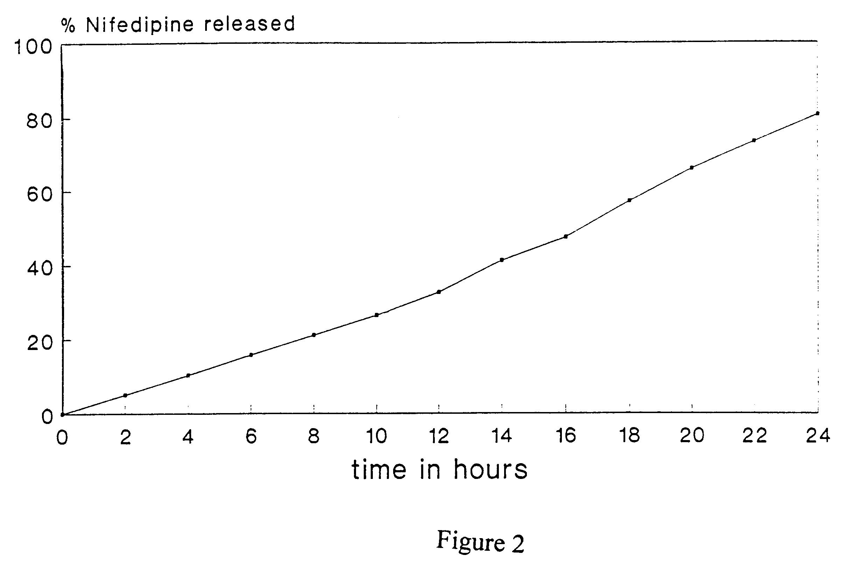

The percentages of release in simulated intestinal fluid of the Table 3a is represented by Figure 2 and is in form of a linear curve of 0^{th} order.

The mean values for the plasma levels over a period of 48 hours in eighteen healthy human test subjects after p.o. administration of the 40 mg tablet are given in Table 4.

**Table 4**

| Mean values (ng/ml) for plasma levels after a single administration of 40 mg nifedipine of Example 8 | |
|---|---|
| time h | Example 8 40 mg |
| 0.0 | 0.00 |
| 0.5 | 1.20 |
| 1.0 | 4.60 |
| 2.0 | 9.50 |
| 2.5 | 11.20 |
| 3.0 | 12.00 |
| 4.0 | 13.60 |
| 6.0 | 15.70 |
| 8.0 | 14.00 |
| 10.0 | 13.90 |
| 12.0 | 15.00 |
| 24.0 | 13.30 |
| 26.0 | 10.10 |
| 28.0 | 8.40 |
| 34.0 | 3.70 |
| 36.0 | 2.70 |
| 48.0 | 1.10 |

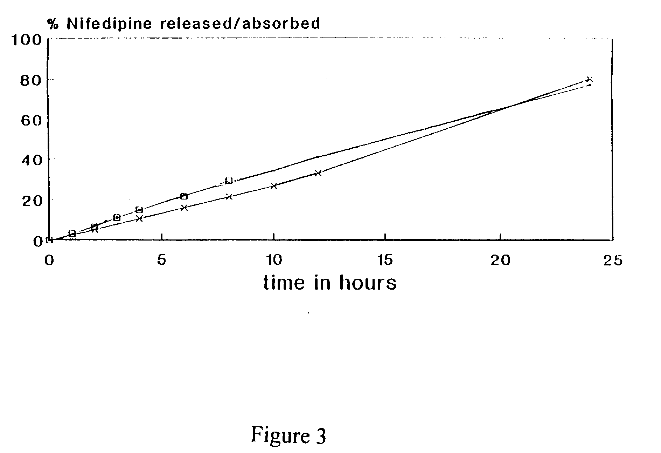

The in vitro/in vivo correlation of released and absorbed (calculated according to Wagner-Nelson, J. pharm. Sci. 52, Vol.6, 1963, page 610-611), nifedipine from the tablet of Example 8 is shown in Figure 3.

### Example 9: Tablets containing 40 mg of nitrendipine

For the preparation of 3,000 tablets, 30.0 g of HPMC 2208/100 cps (Methocel K100LV), 120 g of nitrendipine, 120 g of a commercially available unitary product consisting of 75 % lactose and 25 % powdered cellulose (Cellactose, Meggle) and 145.50 g of microcrystalline cellulose are mixed together.

For a ready-to-compress mixture, 4.5 g of magnesium stearate and 2.25 g of grinded colloidal silicon dioxide are separately added and the mixture is compressed to form tablets weighing 140.75 mg and having a diameter of 7.0 mm, a thickness of 4 mm and a hardness of 80 N.

The tablets may be provided with the coating described in Example 3.

The release values of the cores and of the film-coated tablets in simulated gastric fluid are the same and are given in Table 5.

### Example 10: Tablets containing 40 mg of nitrendipine

For the preparation of 3,000 tablets, 15.0 g of HPMC 2208/100 cps (Methocel K100LV), 120 g of nitrendipine, 135 g of a commercially available unitary product consisting of 75 % lactose and 25 % powdered cellulose (Cellactose, Meggle) and 145.50 g of microcrystalline cellulose are mixed together.

For a ready-to-compress mixture, 4.5 g of magnesium stearate and 2.25 g of grinded colloidal silicon dioxide are separately added and the mixture is compressed to form tablets weighing 140.75 mg and having a diameter of 7.0 mm, a thickness of 4 mm and a hardness of 80 N.

The tablets may be provided with the coating described in Example 3.

The release values of the cores and of the film-coated tablets in simulated gastric fluid are the same and are given in Table 5.

**Table 5**

| Rates of release (mg/h) of nitrendipine in simulated gastric fluid | | | | |
|---|---|---|---|---|
| time | Example 9 | | Example 10 | |
| h | mg/h | % | mg/h | % |
| 0 | 0.00 | 0.0 | 0.00 | 0.0 |
| 2 | 0.37 | 3.7 | 0.40 | 4.0 |
| 4 | 0.37 | 7.5 | 0.49 | 8.9 |
| 6 | 0.36 | 11.1 | 0.50 | 14.0 |
| 8 | 0.38 | 15.0 | 0.48 | 18.8 |
| 10 | 0.35 | 18.5 | 0.44 | 23.2 |
| 12 | 0.32 | 21.7 | 0.40 | 27.6 |
| 14 | 0.29 | 24.6 | 0.35 | 30.6 |
| 16 | 0.26 | 27.2 | 0.32 | 33.8 |
| 18 | 0.23 | 29.5 | 0.31 | 36.9 |
| 20 | 0.20 | 31.4 | 0.30 | 39.8 |
| 22 | 0.20 | 33.4 | 0.26 | 42.5 |
| 24 | 0.19 | 35.3 | 0.28 | 45.3 |

## Claims

1. A pharmaceutical dosage form having prolonged release of zero order of the active ingredient, suitable for maintaining a therapeutically effective plasma level over a period of 24 hours with a once-daily oral administration, containing as active ingredient a therapeutically effective amount of a crystalline, sparingly water-soluble calcium antagonist of the dihydropyridine type, characterised by
a homogeneous matrix containing 5 - 60 % by weight of a crystalline, sparingly water-soluble calcium antagonist of the dihydropyridine type having a specific surface area of from 0.2 to 0.5 m²/g,
2 - 50 % by weight of hydroxypropylmethylcellulose having a molecular weight in the range of about 20,000 - 250,000,
optionally 2 - 25 % by weight of pharmaceutically acceptable excipients controlling release, and
optionally other pharmaceutically acceptable excipients making up the weight of the dosage form to 100 %.

2. A pharmaceutical dosage form according to claim 1 having prolonged release of zero order of an active ingredient upon once-daily oral administration, containing
as active ingredient a therapeutically effective amount of a crystalline dihydropyridine calcium antagonist having a solubility in water of from 0.0001 to 0.01 %,
a homogeneous matrix consisting of 5 - 60 % by weight of the calcium antagonist,
an hydroxypropylmethylcellulose of the 2208 USP XXII class having a molecular weight in the range of about 20,000 - 250,000 in an amount sufficient to prolong the zero order release of the calcium antagonist and maintain a constant therapeutically effective plasma level over a period of 24 hours, said amount being from 2 - 50 % by weight, and
optionally 2 - 25 % by weight of pharmaceutically acceptable excipients controlling release, said excipients being solid polyethylene glycols, polyvinylpyrrolidones, vinylpyrroli-done/vinyl acetate copolymers, pharmaceutically acceptable derivatives of vegetable fats in solid tabletable form having a melting point of above 60° C, and
optionally other pharmaceutically acceptable excipients making up the weight of the dosage form to 100 %, said other excipients being fillers, lubricants and flow regulating agents.

3. Pharmaceutical dosage form according to claim 1, wherein the calcium antagonist is nifedipine.

4. Pharmaceutical dosage form according to claim 1, wherein the calcium antagonist is nitrendipine.

5. Pharmaceutical dosage form according to claim 1, wherein the calcium antagonist is nimodipine, isradipine, nicardipine, niludipine, nigludipine, nisoldipine, felodipine, amlodipine or lacidipine.

6. Pharmaceutical dosage form according to claim 1, wherein the hydroxypropylmethylcellulose has a molecular weight of 20,000 - 120,000.

7. Pharmaceutical dosage form according to claim 1, wherein the dosage form contains 20, 30, 40, 50, 60, 70, 80, 90, 100 or 120 mg of active ingredient.

8. Pharmaceutical dosage form according to claim 1, wherein in vivo the active ingredient is absorbed over a period of approximately 24 hours and the rate of absorption is virtually constant over a period of up to 24 hours.

9. Pharmaceutical dosage form according to claim 1, wherein in vivo the release of the active ingredient has a delayed onset, characterized in that the delayed onset is caused by a film coating having retarding action.

10. A pharmaceutical dosage form according to claim 1 having prolonged release of an active ingredient upon once-daily oral administration, containing as active ingredient crystalline nifedipine, wherein the dosage form is a tablet and comprises:
(a) 40 mg of nifedipine;
(b) 2 - 35 mg of hydroxypropylmethylcellulose type 2208 USP XXII of 100 or 4000 cps;
(c) 20 - 50 mg of lactose and 35 to 60 mg of powdered or microcristalline cellulose or both; or instead 50- 80 mg of a mixture of 75 % of lactose and 25 % of powdered cellulose;
(d) optionally 10 mg of hardened vegetable oil;
(e) 1.5 mg of magnesium stearate; and
(f) optionally 0,25 - 1 mg of highly dispersed silicon dioxide,
so that cores prepared therefrom weigh 140 - 155 mg.

11. A pharmaceutical dosage form acording to claim 1 having prolonged release of an active ingredient upon once-daily oral administration, containing as active ingredient crystalline nitrendipine, wherein the dosage form is a tablet and comprises:
(a) 40 mg of nitrendipine;
(b) 2 - 35 mg of hydroxypropylmethylcellulose of the 2208 USP XXII of 100 or 4000 cps;
(c) 20 - 50 mg of lactose and 35 to 60 mg of powdered or microcristalline cellulose or both; or instead 50 - 80 mg of a mixture of 75 % of lactose and 25 % of powdered cellulose;
(d) optionally 10 mg of hardened vegetable oil;
(e) 1.5 mg of magnesium stearate; and
(f) optionally 0,25 - 1 mg of highly dispersed silicon dioxide,
so that cores prepared therefrom weigh 140 - 155 mg.

12. Pharmaceutical dosage form according to claim 10, wherein the core is provided with a coating comprising :
(a) 1.5 - 2.5 mg of titanium oxide;
(b) 0.5 - 1.5 mg of red iron oxide;
(c) 1 - 3.0 mg of talc;
(d) 2.0 mg of hydroxypropylmethylcellulose type 2910 USP XXII having a viscosity of 5 to 15 cps;
(e) optionally 1 - 1.5 mg of polyethylene glycol 400;
(f) optionally 0.005 mg of polysorbate 80;
(g) optionally 0,5 - 0.75 mg of triethyl citrate; and
(h) optionally 0.5 - 1.5 mg of Eudragit RL dry substance, and/or optionally 0.5 - 1.5 mg of Eudragit Rs dry substance.

13. Pharmaceutical dosage form according to claim 11, wherein the core is provided with a coating comprising :
(a) 1.5 - 2.5 mg of titanium oxide;
(b) 0.5 - 1.5 mg of red iron oxide;
(c) 1 - 3.0 mg of talc;
(d) 2.0 mg of hydroxypropylmethylcellulose type 2910 USP XXII having a viscosity of 5 to 15 cps;
(e) optionally 1 - 1.5 mg of polyethylene glycol 400;
(f) optionally 0.005 mg of polysorbate 80;
(g)optionally 0,5 - 0.75 mg of triethyl citrate; and
(h) optionally 0.5 - 1.5 mg of Eudragit RL dry substance, and/or optionally 0.5 - 1.5 mg of Eudragit Rs dry substance.

14. A pharmaceutical dosage form according to claim 1 having prolonged release of an active ingredient upon once-daily oral administration, containing as active ingredient crystalline nifedipine, wherein the dosage form comprises:
(a) a core containing
(i) 40 mg of nifedipine
(ii) 20 mg of hydroxypropylmethylcellulose of the 2208 USP XXII class having a molecular weight in the range of about 20,000-250,000 and having a viscosity of 4000 cps;
(iii) 40 mg of a mixture of 75 % of lactose and 25 % of powdered cellulose;
(iv) 48.5 mg of microcristalline cellulose;
(v) 1.5 mg of magnesium stearate; and
(b) a film coating for the core containing
(i) 1 part by weight of hydroxypropylmethylcellulose of the 2910 USP XXII type having a viscosity of 5 mPas;
(ii) 0.55 parts by weight of polyethylene glycol 400;
(iii) 1 part by weight of titanium dioxide;
(iv) 0.45 part by weight of red iron oxide;
(v) 0.50 parts by weight of talc.

15. Pharmaceutical dosage form according to claim 14 wherein the nifedipine has a specific surface area of 0.3-0.4 m²/g.

16. A pharmaceutical dosage form according to claim 1 having prolonged release of an active ingredient upon once-daily oral administration, containing as active ingredient, crystalline nifedipine, wherein the dosage form comprises:
(a) a core containing
(i) 40 mg of nifedipine);
(ii) 20 mg of hydroxypropylmethylcellulose of the 2208 USP XXII class having a molecular weight in the range of about 20,000-250,000 and having a viscosity of 4000 cps;
(iii) 40 mg of a mixture of 75 % of lactose and 25 % of powdered cellulose;
(iv) 48.5 mg of microcristalline cellulose;
(v) 1.5 mg of magnesium stearate; and
(vi) 0.75 mg of colloidal silicon dioxide; and
(b) a film coating for the core containing
(i) 1 part by weight of hydroxypropylmethylcellulose of the 2910 USP XXII type having a viscosity of 15 cps;
(ii) 0.55 parts by weight of polyethylene glycol 400;
(iii) 1 part by weight of titanium dioxide;
(iv) 0.45 part by weight of red iron oxide;
(v) 0.50 parts by weight of talc; and
(vi) 0.04 parts by weight of polyethylene glycol 6000.

17. Pharmaceutical dosage form according to claim 16 wherein the nifedipine has a specific surface area of 0.3-0.4 m²/g.

18. Pharmaceutical dosage form according to claim 1, wherein the dosage form is provided with a coating having no retarding action, comprising a film-former, a pigment, an antiadhesive agent and a plasticizer.

19. Pharmaceutical dosage form according to claim 1, wherein the dosage form is provided with a coating producing a lag time, comprising a water-insoluble but water-permeable polymer.

20. A process for the preparation of a pharmaceutical dosage form according to claim 1 characterized in that the dosage form is prepared in a conventional manner.

21. A method for preparing a pharmaceutical dosage form according to claim 1 for the treatment of the indications customary for calcium antagonists of the dihydropyridine typ, comprising the once-daily oral administration thereof to a patient, which method is of conventional manner.

## Patentansprüche

1. Eine pharmazeutische Darreichungsform mit verlängerter Freigabe nullter Ordnung der Wirksubstanz, geeignet für die Aufrechthaltung eines therapeutisch wirksamen Plasmaspiegels während 24 Stunden bei einmal täglicher oraler Verabreichung, enthaltend als Wirksubstanz eine therapeutisch wirksame Menge eines kristallinen, in Wasser schwerlöslichen Calciumantagonisten vom Dihydropyridintyp, gekennzeichnet durch
eine homogene Matrix, enthaltend 5 - 60 Gewichtsprozente eines kristallinen, in Wasser schwerlöslichen Calciumantagonisten vom Dihydropyridintyp mit einer spezifischen Oberfläche von 0.2 bis 0.5 m²/g,
2 - 50 Gewichtsprozente Hydroxypropylmethylcellulose mit einem Molekulargewicht im Bereich von ca. 20,000-250,000,
gegebenenfalls 2 - 25 Gewichtsprozente pharmazeutisch annehmbare, die Freisetzung kontrollierende Hilfsstoffe und
gegebenenfalls andere pharmazeutisch annehmbare Hilfsstoffe, welche das Gewicht der Darreichungsform auf 100 % ergänzen.

2. Eine pharmazeutische Darreichungsform nach Anspruch 1 mit verlängerter Freigabe nullter Ordnung einer Wirksubstanz, nach einmal täglicher Verabreichung, enthaltend
als Wirksubstanz eine therapeutisch wirksame Menge eines kristallinen Dihydropyridin-Calciumantagonisten mit einer Wasserlöslichkeit von 0.0001 bis 0.01 %,
eine homogene Matrix, bestehend aus 5 - 60 Gewichtsprozenten des Calciumantagonisten,
eine Hydroxypropylmethylcellulose der 2208 USP XXII Klasse mit einem Molekulargewicht im Bereich von ca. 20,000-250,000, in einer genügenden Menge, um die Freigabe nullter Ordnung des Calciumantagonisten zu verlängern und einen konstanten, therapeutischen Plasmaspiegel während einer Periode von 24 Stunden aufrechtzuhalten, wobei besagte Menge von 2 - 50 Gewichtsprozente beträgt und
gegebenenfalls 2 - 25 Gewichtsprozente pharmazeutisch annehmbare, die Freisetzung kontrollierende Hilfsstoffe, welche besagten Hilfsstoffe feste Polyethylenglykole, Polyvinylpyrrolidone, Vinylpyrrolidon/Vinylacetat-Copolymere, pharmazeutisch annehmbare Derivate von pflanzlichen Fetten in fester, tablettierbarer Form mit einem Schmelzpunkt von über 60° C sind und
gegebenenfalls andere pharmazeutisch annehmbare Hilfsstoffe, welche das Gewicht der Darreichungsform auf 100 % ergänzen, welche besagten anderen Hilfsstoffe Füllstoffe, Schmiermittel und Fliessreguliermittel sind.

3. Pharmazeutische Darreichungsform nach Anspruch 1, worin der Calciumantagonist Nifedipin ist.

4. Pharmazeutische Darreichungsform nach Anspruch 1, worin der Calciumantagonist Nitrendipin ist.

5. Pharmazeutische Darreichungsform nach Anspruch 1, worin der Calciumantagonist Nimodipin, Isradipin, Nicardipin, Niludipin, Nigludipin, Nisoldipin, Felodipin, Amlodipin oder Lacidipin ist.

6. Pharmazeutische Darreichungsform nach Anspruch 1, worin die Hydroxypropylmethylcellulose ein Molekulargewicht von 20,000 - 120,000 hat.

7. Pharmazeutische Darreichungsform nach Anspruch 1, worin die Darreichungsform 20, 30, 40, 50, 70, 60, 80, 90, 100 oder 120 mg Wirkstoff enthält.

8. Pharmazeutische Darreichungsform nach Anspruch 1, worin in vivo die Wirksubstanz über eine Periode von annähernd 24 Stunden absorbiert wird und die Absorptionsrate über eine Periode von bis zu 24 Stunden praktisch konstant verläuft.

9. Pharmazeutische Darreichungsform nach Anspruch 1, worin in vivo die Freisetzung des Wirkstoffes verzögert beginnt, dadurch gekennzeichnet, dass die verzögerte Freigabe durch einen Filmüberzug mit verzögernder Wirkung verursacht wird.

10. Eine pharmazeutische Darreichungsform nach Anspruch 1 mit verlängerter Freigabe einer Wirksubstanz nach einmal täglicher Verabreichung, enthaltend als Wirksubstanz kristallines Nifedipin, worin die Darreichungsform eine Tablette ist und enthält:
(a) 40 mg Nifedipin;
(b) 2 - 35 mg Hydroxypropylmethylcellulose Typ 2208 USP XXII von 100 oder 4000 cps;
(c) 20 - 50 mg Lactose und 35 bis 60 mg pulverisierte oder mikrokristalline Cellulose, oder beide; oder anstelle davon etwa 50 - 80 mg eines Gemisches aus 75 % Lactose und 25 % pulverisierter Cellulose;
(d) gegebenenfalls etwa 10 mg gehärtetes Pflanzenöl;
(e) 1.5 mg Magnesiumstearat; und
(f) gegebenenfalls 0,25 - 1 mg hochdisperses Siliciumdioxid; so dass daraus hergestellte Kerne 140 - 155 mg wiegen.

11. Eine pharmazeutische Darreichungsform nach Anspruch 1 mit verlängerter Freigabe einer Wirksubstanz nach einmal täglicher Verabreichung, enthaltend als Wirksubstanz kristallines Nitrendipin, worin die Darreichungsform eine Tablette ist und enthält:
(a) 40 mg Nitrendipin;
(b) 2 - 35 mg Hydroxypropylmethylcellulose Typ 2208 USP XXII von 100 oder 4000 cps;
(c) 20 - 50 mg Lactose und 35 bis 60 mg pulverisierte oder mikrokristalline Cellulose, oder beide; oder anstelle davon etwa 50 - 80 mg eines Gemisches aus 75 % Lactose und 25 % pulverisierter Cellulose;
(d) gegebenenfalls etwa 10 mg gehärtetes Pflanzenöl;
(e) 1.5 mg Magnesiumstearat;
(f) gegebenenfalls 0,25 - 1 mg hochdisperses Siliciumdioxid; so dass daraus hergestellte Kerne 140 - 155 mg wiegen.

12. Pharmazeutische Darreichungsform nach Anspruch 10, worin der Kern mit einem Überzug versehen ist enthaltend:
(a) 1.5 - 2.5 mg Titanoxid;
(b) 0.5 - 1.5 mg Eisenoxidrot;
(c) 1 - 3.0 mg Talk;
(d) 2.0 mg Hydroxypropylmethylcellulose Typ 2910 USP XXII mit einer Viskosität von 5 bis 15 cps;
(e) gegebenenfalls 1 - 1.5 mg Polyethylenglykol 400;
(f) gegebenenfalls 0.005 mg Polysorbat 80;
(g) gegebenenfalls 0.5 - 0.7 mg Triethylcitrat und
(h) gegebenenfalls 0.5 - 1.5 mg Eudragit RL Trockensubstanz, und/oder gegebenenfalls 0.5 - 1.5 mg Eudragit RS Trockensubstanz.

13. Pharmazeutische Darreichungsform nach Anspruch 11, worin der Kern mit einem Überzug versehen ist enthaltend:
(a) 1.5 - 2.5 mg Titanoxid;
(b) 0.5 - 1.5 mg Eisenoxidrot;
(c) 1 - 3.0 mg Talk;
(d) 2.0 mg Hydroxypropylmethylcellulose Typ 2910 USP XXII mit einer Viskosität von 5 bis 15 cps;
(e) gegebenenfalls 1 - 1.5 mg Polyethylenglykol 400;
(f) gegebenenfalls 0.005 mg Polysorbat 80;
(g) gegebenenfalls 0,5 - 0.7 mg Triethylcitrat und
(h) gegebenenfalls 0.5 - 1.5 mg Eudragit RL Trockensubstanz und/oder gegebenenfalls 0.5 - 1.5 mg Eudragit RS Trockensubstanz.

14. Eine pharmazeutische Darreichungsform nach Anspruch 1 mit verlängerter Freigabe der Wirksubstanz nach einmal täglicher Verabreichung, enthaltend als Wirksubstanz kristallines Nifedipin, worin die Darreichungsform enthält:
(a) einen Kern enthaltend
(i) 40 mg Nifedipin;
(ii) 20 mg Hydroxypropylmethylcellulose Typ 2208 USP XXII mit einem Molekulargewicht im Bereich von etwa 20,000-250,000 und einer Viskosität von 4000 cps;
(iii) 40 mg eines Gemisches aus 75 % Lactose und 25 % pulverisierter Cellulose;
(iv) 48.5 mg mikrokristalline Cellulose;
(v) 1.5 mg Magnesiumstearat; und
(b) einen Filmüberzug für den Kern enthaltend
(i) 1 Gewichtsteil Hydroxypropylmethylcellulose Typ 2910 USP XXII mit einer Viskosität von 5 mPas;
(ii) 0.55 Gewichtsteile Polyethylenglykol 400;
(iii) 1 Gewichtsteil Titandioxid;
(iv) 0.45 Gewichtsteile Eisenoxidrot;
(v) 0.50 Gewichtsteile Talk.

15. Pharmazeutische Darreichungsform nach Anspruch 14, worin Nifedipin eine spezifische Oberfläche von 0.3 - 0.4 m²/g hat.

16. Eine pharmazeutische narreichungsform nach Anspruch 1 mit verlängerter Freigabe der Wirksubstanz nach einmal täglicher Verabreichung, enthaltend als Wirksubstanz kristallines Nifedipin, worin die Darreichungsform enthält:
(a) einen Kern enthaltend
(i) 40 mg Nifedipin;
(ii) 20 mg Hydroxypropylmethylcellulose Typ 2208 USP XXII mit einem Molekulargewicht im Bereich von etwa 20,000-250,000 und einer Viskosität von 4000 cps;
(iii) 40 mg eines Gemisches aus 75 % Lactose und 25 % pulverisierter Cellulose;
(iv) 48.5 mg mikrokristalline Cellulose;
(v) 1.5 mg Magnesiumstearat; und
(vi) 0.75 mg kolloidales Siliciumdioxid; und
(b) einen Filmüberzug für den Kern enthaltend
(i) 1 Gewichtsteil Hydroxypropylmethylcellulose Typ 2910 USP XXII mit einer Viskosität von 15 cps;
(ii) 0.55 Gewichtsteile Polyethylenglykol 400;
(iii) 1 Gewichtsteil Titandioxid;
(iv) 0.45 Gewichtsteile Eisenoxidrot;
(v) 0.50 Gewichtsteile Talk; und
(vi) 0.04 Gewichtsteile Polyethylenglykol 6000.

17. Pharmazeutische Darreichungsform nach Anspruch 16, worin Nifedipin eine spezifische Oberfläche von 0.3 - 0.4 m²/g hat.

18. Pharmazeutische Darreichungsform nach Anspruch 1, worin die Darreichungsform mit einem Überzug ohne verzögernde Wirkung versehen ist, enthaltend einen Filmbildner, ein Pigment, ein Antiklebemittel und einen Weichmacher.

19. Pharmazeutische Darreichungsform nach Anspruch 1, worin die Darreichungsform mit einem Überzug versehen ist, der eine verzögernde Wirkung besitzt, enthaltend ein wasser-unlösliches aber wasser-durchlässiges Polymer

20. Ein Verfahren zur Herstellung einer pharmazeutischen Darreichungsform gemäss Anspruch 1, dadurch gekennzeichnet, dass man die Darreichungsform auf konventionelle Weise herstellt.

21. Eine Methode zur Herstellung einer pharmazeutischen Darreichungsform gemäss Anspruch 1 zur Behandlung der für Calciumantagonisten vom Dihydropyridintyp üblichen Indikationen, umfassend deren einmal tägliche orale Verabreichung an einen Patienten, welche Methode von konventioneller Weise ist.

## Revendications

1. Forme posologique pharmaceutique permettant une libération prolongée d'ordre zéro de l'ingrédient actif, appropriée pour maintenir un taux plasmatique thérapeutiquement efficace pendant une période de 24 heures en administration par voie orale avec une seule prise quotidienne, contenant comme ingrédient actif une quantité thérapeutiquement efficace d'un antagoniste du calcium, cristallin et faiblement soluble dans l'eau, du type des dihydropyridines, caractérisée par
une matrice homogène contenant de 5 à 60 % en poids d'un antagoniste du calcium, cristallin et faiblement soluble dans l'eau, du type des dihydropyridines, présentant une aire spécifique de 0,2 à 0,5 m²/g,
de 2 à 50% en poids d'une hydroxypropylméthylcellulose dont la masse moléculaire vaut d'environ 20 000 à 250 000,
éventuellement de 2 à 25 % en poids d'excipients pharmaceutiquement acceptables régulant la libération,
et éventuellement d'autres excipients pharmaceutiquement acceptables complétant à 100 % le poids de la forme posologique.

2. Forme posologique pharmaceutique conforme à la revendication 1, permettant une libération prolongée d'ordre zéro d'un ingrédient actif en administration par voie orale avec une seule prise quotidienne, contenant :
comme ingrédient actif, une quantité thérapeutiquement efficace d'un antagoniste du calcium cristallin, du type des dihydropyridines, dont la solubilité dans l'eau vaut de 0,0001 à 0,01 %,
une matrice homogène constituée de 5 à 60 % en poids de l'antagoniste du calcium,
une hydroxypropylméthylcellulose de la classe 2208 USP XXII dont la masse moléculaire vaut d'environ 20 000 à 250 000, en une proportion suffisante pour prolonger la libération d'ordre zéro de l'antagoniste du calcium et pour maintenir un taux plasmatique constant et thérapeutiquement efficace pendant une période de 24 heures, ladite proportion valant de 2 à 50 % en poids, et
éventuellement, 2 à 25 % en poids d'excipients pharmaceutiquement acceptables régulant la libération, ces excipients étant des polyéthylèneglycols solides, des polyvinylpyrrolidones, des copolymères de vinylpyrrolidone et d'acétate de vinyle, des dérivés pharmaceutiquement acceptables de graisses végétales, sous forme solide et pouvant être mise en comprimés, et dont le point de fusion est supérieur à 60°C, et
éventuellement, d'autres excipients pharmaceutiquement acceptables complétant à 100 % le poids de la forme posologique, ces autres excipients étant des charges, des lubrifiants et des agents de régulation de l'écoulement.

3. Forme posologique pharmaceutique conforme à la revendication 1, dans laquelle l'antagoniste du calcium est de la nifédipine.

4. Forme posologique pharmaceutique conforme à la revendication 1, dans laquelle l'antagoniste du calcium est de la nitrendipine.

5. Forme posologique pharmaceutique conforme à la revendication 1, dans laquelle l'antagoniste du calcium est de la nimodipine, de l'isradipine, de la nicardipine, de la niludipine, de la nigludipine, de la nisoldipine, de la félodipine, de l'amlodipine ou de la lacidipine.

6. Forme posologique pharmaceutique conforme à la revendication 1, dans laquelle la masse moléculaire de l'hydroxypropylméthylcellulose vaut de 20 000 à 120 000.

7. Forme posologique pharmaceutique conforme à la revendication 1, laquelle forme posologique contient 20, 30, 40, 50, 60, 70, 80, 90, 100 ou 120 mg d'ingrédient actif.

8. Forme posologique pharmaceutique conforme à la revendication 1, avec laquelle l'ingrédient actif est absorbé in vivo au cours d'un laps de temps d'environ 24 heures, la vitesse d'absorption étant pratiquement constante pendant un laps de temps allant jusqu'à 24 heures.

9. Forme posologique pharmaceutique conforme à la revendication 1, avec laquelle la libération in vivo de l'ingrédient actif commence avec un certain retard, caractérisée en ce que ce retard est provoqué par un film d'enrobage qui exerce une action de retardement.

10. Forme posologique pharmaceutique conforme à la revendication 1, permettant une libération prolongée d'un ingrédient actif en administration par voie orale avec une seule prise quotidienne, contenant de la nifédipine cristalline comme ingrédient actif, laquelle forme posologique est un comprimé qui comprend :
a) 40 mg de nifédipine ;
b) 2 à 35 mg d'hydroxypropylméthylcellulose, type 2208 USP XXII, présentant une viscosité de 100 ou 4000 centipoises ;
c) 20 à 50 mg de lactose et 35 à 60 mg de cellulose microcristalline ou en poudre, ou des deux types ; ou bien, à la place, 50 à 80 mg d'un mélange de 75 % de lactose et de 25 % de cellulose en poudre ;
d) éventuellement, 10 mg d'une huile végétale durcie ;
e) 1,5 mg de stéarate de magnésium ;
et
f) éventuellement, 0,25 à 1 mg de dioxyde de silicium très dispersé ;
de telle sorte que les noyaux qu'on prépare avec ces ingrédients pèsent de 140 à 155 mg.

11. Forme posologique pharmaceutique conforme à la revendication 1, permettant une libération prolongée d'un ingrédient actif en administration par voie orale avec une seule prise quotidienne, contenant de la nitrendipine cristalline comme ingrédient actif, laquelle forme posologique est un comprimé qui comprend :
a) 40 mg de nitrendipine ;
b) 2 à 35 mg d'hydroxypropylméthylcellulose, type 2208 USP XXII, présentant une viscosité de 100 ou 4000 centipoises ;
c) 20 à 50 mg de lactose et 35 à 60 mg de cellulose microcristalline ou en poudre, ou des deux types ; ou bien, à la place, 50 à 80 mg d'un mélange de 75 % de lactose et de 25 % de cellulose en poudre ;
d) éventuellement, 10 mg d'une huile végétale durcie :
e) 1,5 mg de stéarate de magnésium ;
et
f) éventuellement, 0,25 à 1 mg de dioxyde de silicium très dispersé ;
de telle sorte que les noyaux qu'on prépare avec ces ingrédients pèsent de 140 à 155 mg.

12. Forme posologique pharmaceutique conforme à la revendication 10, dans laquelle le noyau est muni d'un enrobage comprenant :
a) 1,5 à 2,5 mg d'oxyde de titane ;
b) 0,5 à 1,5 mg d'oxyde rouge de fer ;
c) 1 à 3,0 mg de talc ;
d) 2,0 mg d'hydroxypropylméthylcellulose, type 2910 USP XXII, présentant une viscosité de 5 à 15 centipoises ;
e) éventuellement, 1 à 1,5 mg de polyéthylèneglycol 400 ;
f) éventuellement, 0,005 mg de polysorbate 80 ;
g) éventuellement, 0,5 à 0,75 mg de citrate de triéthyle ;
et
h) éventuellement, 0,5 à 1,5 mg de substance sèche Eudragit RL, et/ou éventuellement, 0,5 à 1,5 mg de substance sèche Eudragit Rs.

13. Forme posologique pharmaceutique conforme à la revendication 11, dans laquelle le noyau est muni d'un enrobage comprenant :
a) 1,5 à 2,5 mg d'oxyde de titane ;
b) 0,5 à 1,5 mg d'oxyde rouge de fer ;
c) 1 à 3,0 mg de talc ;
d) 2,0 mg d'hydroxypropylméthylcellulose, type 2910 USP XXII, présentant une viscosité de 5 à 15 centipoises ;
e) éventuellement, 1 à 1,5 mg de polyéthylèneglycol 400 ;
f) éventuellement, 0,005 mg de polysorbate 80 ;
g) éventuellement, 0,5 à 0,75 mg de citrate de triéthyle ;
et
h) éventuellement, 0,5 à 1,5 mg de substance sèche Eudragit RL, et/ou éventuellement, 0,5 à 1,5 mg de substance sèche Eudragit Rs.

14. Forme posologique pharmaceutique conforme à la revendication 1, permettant une libération prolongée d'un ingrédient actif en administration par voie orale avec une seule prise quotidienne, contenant de la nifédipine cristalline comme ingrédient actif, laquelle forme posologique comprend :
a) un noyau contenant :
1) 40 mg de nifédipine ;
2) 20 mg d'hydroxypropylméthylcellulose, de classe 2208 USP XXII, dont la masse moléculaire vaut d'environ 20 000 à 250 000 et dont la viscosité vaut 4000 centipoises ;
3) 40 mg d'un mélange de 75 % de lactose et de 25 % de cellulose en poudre ;
4) 48,5 mg de cellulose microcristalline ;
et
5) 1,5 mg de stéarate de magnésium ;
et
b) un film enrobant le noyau et contenant :
1) 1 partie en poids d'hydroxypropylméthylcellulose, type 2910 USP XXII, présentant une viscosité de 5 mPa.s ;
2) 0,55 partie en poids de polyéthylèneglycol 400 ;
3) 1 partie en poids de dioxyde de titane ;
4) 0,45 partie en poids d'oxyde rouge de fer ;
et
5) 0,50 partie en poids de talc.

15. Forme posologique pharmaceutique conforme à la revendication 14, dans laquelle l'aire spécifique de la nifédipine vaut de 0,3 à 0,4 m²/g.

16. Forme posologique pharmaceutique conforme à la revendication 1, permettant une libération prolongée d'un ingrédient actif en administration par voie orale avec une seule prise quotidienne, contenant de la nifédipine cristalline comme ingrédient actif, laquelle forme posologique comprend :
a) un noyau contenant :
1) 40 mg de nifédipine ;
2) 20 mg d'hydroxypropylméthylcellulose, de classe 2208 USP XXII, dont la masse moléculaire vaut d'environ 20 000 à 250 000 et dont la viscosité vaut 4000 centipoises ;
3) 40 mg d'un mélange de 75 % de lactose et de 25 % de cellulose en poudre ;
4) 48,5 mg de cellulose microcristalline ;
5) 1,5 mg de stéarate de magnésium ;
et
6) 0,75 mg de dioxyde de silicium colloïdal ;
et
b) un film enrobant le noyau et contenant :
1) 1 partie en poids d'hydroxypropylméthylcellulose, type 2910 USP XXII, présentant une viscosité de 15 centipoises ;
2) 0,55 partie en poids de polyéthylèneglycol 400 ;
3) 1 partie en poids de dioxyde de titane ;
4) 0,45 partie en poids d'oxyde rouge de fer ;
5) 0,50 partie en poids de talc ;
et
6) 0,04 partie en poids de polyéthylèneglycol 6000.

17. Forme posologique pharmaceutique conforme à la revendication 16, dans laquelle l'aire spécifique de la nifédipine vaut de 0,3 à 0,4 m²/g.

18. Forme posologique pharmaceutique conforme à la revendication 1, laquelle forme posologique est munie d'un enrobage qui n'exerce pas d'action de retardement et qui comprend un agent formateur de film, un pigment, un agent anti-adhésif et un plastifiant.

19. Forme posologique pharmaceutique conforme à la revendication 1, laquelle forme posologique est munie d'un enrobage entraînant un temps de retard, qui comprend un polymère insoluble dans l'eau, mais perméable à l'eau.

20. Procédé de préparation d'une forme posologique pharmaceutique conforme à la revendication 1, caractérisé en ce que la forme posologique est préparée de façon classique.

21. Procédé de préparation d'une forme posologique pharmaceutique conforme à la revendication 1, destinée au traitement des indications habituelles des antagonistes du calcium du type des dihydropyridines, traitement comprenant le fait d'administrer un tel médicament par voie orale à un patient, en une seule prise quotidienne, lequel procédé est de type classique.
